(19) Europäisches Patentamt
European Patent Office
Office européen des brevets

(11) **EP 3 377 181 B1**

(12) **EUROPEAN PATENT SPECIFICATION**

(45) Date of publication and mention
of the grant of the patent:
**17.07.2024 Bulletin 2024/29**

(21) Application number: **15832932.6**

(22) Date of filing: **14.12.2015**

(51) International Patent Classification (IPC):
*A61Q 11/00* (2006.01)  *A61K 8/22* (2006.01)
*A61K 8/27* (2006.01)  *A61K 8/81* (2006.01)

(52) Cooperative Patent Classification (CPC):
**A61Q 11/00; A61K 8/22; A61K 8/27; A61K 8/8176;**
A61K 2800/43

(86) International application number:
**PCT/US2015/065439**

(87) International publication number:
**WO 2017/105381 (22.06.2017 Gazette 2017/25)**

(54) **STABLE WHITENING ORAL CARE DENTIFRICE COMPOSITIONS**

STABILE BLEICHENDE ZAHNPASTENZUSAMMENSETZUNGEN

COMPOSITIONS STABLES DE DENTIFRICE DE SOINS BUCCAUX BLANCHISSANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**26.09.2018 Bulletin 2018/39**

(73) Proprietor: **Colgate-Palmolive Company
New York, NY 10022 (US)**

(72) Inventors:
• **ONTUMI, Dennis
Easton
Pennsylvania 18045 (US)**
• **MALONEY, Venda Porter
Metuchen, NJ 08840 (US)**

• **CHOPRA, Suman
Monroe, NJ 08831 (US)**

(74) Representative: **Wibbelmann, Jobst
Wuesthoff & Wuesthoff
Patentanwälte und Rechtsanwalt PartG mbB
Schweigerstrasse 2
81541 München (DE)**

(56) References cited:
WO-A1-2006/079342    WO-A1-2015/099638
WO-A1-2015/099642    WO-A2-2014/184084
US-A1- 2006 045 854    US-A1- 2007 122 361

• DATABASE GNPD [online] MINTEL; June 2011
(2011-06-01), "Whitening fluoride toothpaste",
XP002757140, Database accession no. 1570282

**Description**

**BACKGROUND**

Whitening agents, such as hydrogen peroxide, are added to oral care compositions to whiten teeth by bleaching extrinsic and intrinsic stains. Many whitening agents, including hydrogen peroxide, act by breaking the chemical bonds that make up the chromophores in the color compounds, which stain the teeth. This action prevents the chromophores from absorbing visible light, resulting in enhanced tooth whiteness. While conventional, whitening oral care compositions are useful for whitening teeth, formulations that achieve greater and/or faster whitening are desired. Furthermore, the high concentrations of whitening agents often present in conventional oral care compositions may cause irritation of soft tissues in the mouth and/or tooth sensitivity, precluding their use in some individuals. In addition, many regions outside of the United States restrict the amount of hydrogen peroxide in toothpastes.

Improvements to conventional whitening oral care compositions have been reported. For example, whitening oral care compositions containing low concentrations of hydrogen peroxide and pigment blue are known in the art. These formulations result in efficacious whitening after only a single use, despite the low levels of hydrogen peroxide present in the formulation. The effective whitening is likely due to the deposition of the blue pigment onto teeth, which allows the off-white or yellow color of teeth to appear whiter. Furthermore, despite the known effects of hydrogen peroxide on chromophores, these pigment-containing formulations, which may include peroxides complexed with polyvinylpyrrolidone polymers, are surprisingly stable.

Notwithstanding these achievements, further improvements to whitening oral care compositions are desired. For example, it is known in the art that salivary enzymes, which are produced by bacteria in the oral cavity, degrade whitening agents such as hydrogen peroxide. *See, e.g.,* Kraus et al. "whole saliva ... decomposed more than 90% of the peroxide" (Journal of Bacteriology, 1957, 76:727, page 733, left column, paragraph 1). Preventing or reducing such degradation may further increase the efficacy of whitening oral care compositions, particularly those containing a low concentration of whitening agent. Accordingly, whitening oral care compositions, capable of prolonged whitening by retaining the activity of whitening agents for a longer period in the oral cavity, remain desired in the art.

WO 2015/099642 A1, US 2007/122361 A1, and WO 2006/079342 A1 disclose whitening dentifrice compositions.

**BRIEF SUMMARY**

The present disclosure is directed to a stable whitening dentifrice composition comprising: a whitening complex, wherein the whitening complex comprises (a) a whitening agent being hydrogen peroxide and (b) a cross-linked vinyl lactam based polymer being a cross-linked polyvinylpyrrolidone, a blue pigment having a blue to blue-violet color with a hue angle in the CIELAB system ranging from 200 degrees to 320 degrees, a zinc salt in an amount ranging from 0.5% to 2% by weight, said zinc salt comprising zinc oxide; and a linear polyvinylpyrrolidone or an additional cross-linked polyvinylpyrrolidone, wherein the additional cross-linked polyvinylpyrrolidone is not complexed with the whitening agent, and an orally acceptable vehicle.

Also provided herein is a method of whitening a tooth surface, the method including: contacting a tooth surface with a stable whitening dentifrice composition including: a whitening complex, wherein the whitening complex includes (a) a whitening agent and (b) a vinyl lactam based polymer, a blue pigment having a blue to blue-violet color with a hue angle in the CIELAB system ranging from 200 degrees to 320 degrees, a zinc salt, and an orally acceptable vehicle.

Further areas of applicability of the present disclosure will become apparent from the detailed description provided hereinafter. It should be understood that the detailed description and specific examples, while indicating typical embodiments, are intended for purposes of illustration only and are not intended to limit the scope of the disclosure.

**DETAILED DESCRIPTION**

The following description of the embodiments is merely exemplary in nature and is in no way intended to limit the disclosure, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Compositions

The present inventors have surprisingly discovered that zinc may be included in a whitening dentifrice composition, which also includes a blue-violet pigment as described herein, to further enhance whitening efficacy. Without being bound by theory, it is believed that zinc inhibits decay of whitening agents, such as hydrogen peroxide, by destroying bacteria in the saliva, which, as noted above, release enzymes that degrade whitening agents. Accordingly, the present oral care dentifrices may have greater whitening efficacy and result in more continuous whitening in comparison to art-known dentifrice compositions. Additionally, due to the enhanced stability of the whitening agents in the oral cavity, the present oral care dentifrices may be formulated with reduced amounts of whitening agents without a concomitant decrease in whitening efficacy. Further, the present compositions are stable, even after 3 months of aging at 40 °C. This stability is particularly surprising given that transition metal elements, such as zinc, are known to increase the decomposition of whitening agents, while the whitening agents, themselves, are known to degrade colored pigments. *See, for example,* Fig. 1d and Fig. 2 of Feng *et al.,* which demonstrate that transition metals may be used to catalyze the oxidation of rhodomine 6G by hydrogen peroxide as indicated by a change in fluorescence (Analytica Chimica Acta, 2002, 455:187-191). Accordingly, the present stable whitening oral care dentifrice compositions exhibit improved properties in comparison to art-known whitening compositions.

*Complexed Whitening Agents*

The present stable whitening oral care dentifrice compositions include a whitening complex. As used herein a "whitening complex" includes a whitening agent as described herein complexed with a polymer or copolymer, which releases the whitening agent upon exposure to highly aqueous environments, such as in the oral cavity. As used herein, a "complex" is an entity formed by a loose association involving two or more molecular entities (ionic or uncharged), *e.g.*, a whitening agent and a polymer.

The term "polymer" as used herein, is given its ordinary meaning as used in the art, *i.e.,* a molecular structure comprising one or more repeat units (monomers), connected by covalent bonds. The repeat units may all be identical, or in some cases, there may be more than one type of repeat unit present within the polymer. If more than one type of repeat unit is present within the polymer, then the polymer is said to be a "copolymer." It is to be understood that in any embodiment employing a polymer, the polymer being employed may be a copolymer. The repeat units forming the copolymer may be arranged in any fashion. For example, the repeat units may be arranged in a random order, in an alternating order, or as a block copolymer, *i.e.,* comprising one or more regions each comprising a first repeat unit (e.g., a first block), and one or more regions each comprising a second repeat unit (e.g., a second block), *etc.* Block copolymers may have two (a diblock copolymer), three (a triblock copolymer), or more numbers of distinct blocks.

The whitening agent associated with a polymer of the present application includes polymers that are typically cross-linked and are capable of absorbing, adsorbing, complexing or otherwise associating with the provided whitening agent of the present application. Further, the polymer is suitably facilitated to retain the whitening agent of the present application. Such retained whitening agent source discharges the whitening agent when it is applied onto the teeth for whitening.

Suitable polymers and co-polymers are N-vinyl lactam based polymers and copolymers. The monomers for preparing a vinyl lactam-based polymer or co-polymer of the present application includes any monomer having 3 to 8 atoms in a heterocyclic ring, comprising a carbonyl carbon atom and a heteroatom (such as N, S, O) in its vinyl moiety.

Suitable monomers include but not limited to N-vinyl-2-pyrrolidone, N-vinyl-2-piperidone, N-vinyl-3-methyl-pyrrolidinone, N-vinyl-3-methyl-piperidone, N-vinyl-3-methylcaprolactam, N-vinyl-4-methyl-pyrrolidinone, N-vinyl-4-methyl-2-pyrrolidone, N-vinyl-4-methyl-piperidone, N-vinyl-4-methyl-caprolactam, N-vinyl-5-methyl-pyrrolidinone, N-vinyl-5-ethyl-2-pyrrolidone, N-vinyl-4-methyl-piperidone, N-vinyl-3-ethyl-pyrrolidinone, N-vinyl-4,5-dimethyl-pyrrolidinone, N-vinyl-5,5-dimethyl-pyrrolidinone, N-vinyl-3,3,5-trimethyl-pyrrolidinone, N-vinyl-5-methyl-5-ethyl-pyrrolidinone, N-vinyl-3,4,5-trimethyl-3-ethyl-pyrrolidinone, N-vinyl-6-methyl-2-piperidone, N-vinyl-6-ethyl-2-piperidone, N-vinyl-3,5-dimethyl-2-piperidone, N-vinyl-4,4-dimethyl-2-piperidone, N-vinyl-2-caprolactam, N-vinyl-7-methyl-caprolactam, N-vinyl-7-ethyl-caprolactam, N-vinyl-3,5-dimethyl-caprolactam, N-vinyl-4,6-dimethyl-caprolactam, N-vinyl-3,5,7-trimethyl-caprolactam, N-vinyl-2-valerolactam, N-vinyl-hexahydro-2-azepinone, N-vinyl-octahydro-2-azocinone, N-vinyl octahydro-2-azoninone, and N-vinyl decahydro-2-azecinone.

The polymer is a cross-linked polyvinylpyrrolidone, also known as poly-N-vinyl-poly-2-pyrrolidone, and commonly abbreviated to cross-linked "PVP." PVP generally refers to a polymer containing vinylpyrrolidone (also referred to as N-vinylpyrrolidone, N-vinyl-2-pyrrolidone and N-vinyl-2-pyrrolidinone) as a monomeric unit. The monomeric unit consists of a polar imide group, four non-polar methylene groups and a non-polar methane group. Cross linked PVP includes those commercially available as Kollidon® and Luvicross®, marketed by BASF, Mount Olive, N.J., USA; and PolyPlasdone® INF-10, marketed by ISP Corporation, Wayne, N.J., USA.

In some embodiments, the cross-linked polyvinylpyrrolidone is complexed with a peroxide whitening agent, such as hydrogen peroxide (hereinafter "PVP-H$_2$0$_2$"). Upon exposure to highly aqueous environments, such as in the oral cavity, the PVP-H$_2$0$_2$ dissociates into individual species (PVP polymer and H$_2$0$_2$). Suitable cross-linked complexes of PVP-H$_2$0$_2$ are known in the art and are disclosed, for example, in U.S. Patent No. 5,122,370. Commercially available complexes of hydrogen peroxide adsorbed to cross-linked polyvinylpyrrolidone include, for example, Peroxydone XL-10 and Peroxydone K-30, marketed by ISP Corporation, Wayne, N.J., USA.

Some embodiments of the present disclosure provide stable whitening oral care dentifrice compositions comprising from about 0.05% to about 25%, by weight cross-linked polyvinylpyrrolidone complexed with the whitening agent. Other embodiments provide stable whitening oral care dentifrice compositions comprising from about 0.1% to about 15%, by weight cross-linked polyvinylpyrrolidone complexed with the whitening agent. Still other embodiments provide stable whitening oral care dentifrice compositions comprising: from about 0.25% to about 10%, by weight cross-linked polyvinylpyrrolidone complexed with the whitening agent. Yet other embodiments provide stable whitening oral care dentifrice compositions comprising from about 0.5% to about 10%, by weight cross-linked polyvinylpyrrolidone complexed with the whitening agent, while other embodiments provide stable whitening oral care dentifrice compositions comprising from about 0.5% to about 8% by weight cross-linked polyvinylpyrrolidone complexed with the whitening agent. In some embodiments, the stable whitening oral care dentifrice compositions comprise about 0.5% to about 5% by weight cross-linked polyvinylpyrrolidone complexed with the whitening agent, or about 0.5% to about 3%, by weight cross-linked polyvinylpyrrolidone complexed with a whitening agent, or about 0.5% to about 2%, by weight cross-linked polyvinylpyrrolidone complexed with the whitening agent, or about 0.05% to 0.55% by weight cross-linked polyvinylpyrrolidone complexed with a whitening agent, such as about 0.055%. In some embodiments, the whitening complex may contain about 1.0-30 wt%, e.g., 15-25 wt%, for example about 17-22 wt% of hydrogen peroxide and about 5-15 wt%, for example about 7-12 wt% total nitrogen, for example, having substantially the same specifications as Peroxydone XL-10, e.g., available from International Specialty Products (Wayne, NX).

In some embodiments, the present stable whitening oral care dentifrice compositions further comprise from about 1% to about 20%, such as about 1% to about 15%, such as about 1% to about 10%, such as about 5% to about 15%, such as about 6% to about 12%, such as about 8% to about 11%, such as about 8.5% to about 11%, such as about 9.9% to about 10%, such as about 5.75% by weight of a cross-linked polymer, such as cross-linked polyvinylpyrrolidone, which is in addition to the cross-linked polymer or co-polymer included in the whitening complex.

In other embodiments, the present stable whitening oral care dentifrice compositions further comprise about 1% to about 20%, such as about 1% to about 15%, such as about 1% to about 10%, such as about 5% to about 15%, such as about 7% to about 12%, such as about 8% to about 11%, such as about 8.5% to about 11%, such as about 9.9% to about 10%, such as about 9% by weight of a linear polymer, such as polyvinylpyrrolidone, *e.g.,* PVP K-Series or Povidone K-30 marketed by AAA International Corp., Downers Grove, Ill., USA; PVP K-30 USP24 and industry grade, PVP VA-64, PVP K-17 and PVP K-90, marketed by Peakchem, Hangzhou, China.

*Pigments*

The present stable whitening oral care dentifrice compositions include a pigment as set forth in claim 1 As used herein, a "pigment" is a synthetic or natural water insoluble substance, which imparts color to another substance. In some embodiments, the pigments further enhance the whiteness of the teeth. As is known in the art, the visual perception of a white substance can be altered through the deposition of an optical brightener, a blue pigment or a blue dye. This effect is commonly used in laundry detergent products to make white clothes appear "whiter" to the human eye. The same concept has been applied to tooth whitening. See PCT Publication No. WO 2015/099642 to Colgate-Palmolive Company.

In some embodiments, the pigment included in the present stable whitening oral care dentifrice compositions should have a hue angle, h, in the CIELAB system ranging from about 220 degrees to about 320 degrees, typically between about 250 degrees and about 290 degrees. As is well known in the art, "CIELAB" is a color measurement system adopted by the Commission Internationale de l'Eclairage (CIE) in 1976. It is based on a three-dimensional color space. The system was developed to represent color in a manner that is consistent with human vision and proportional to perceived color differences. CIELAB values describe the coordinates of a specific color in a three dimensional space. There are three axes: L* describing light to dark, b* for blue to yellow, and a* for red to green. Any point in the three dimensional CIELAB color space can be described by its L* a* and b* coordinates. The same point can also be described by L*, hue angle and chroma, which uses cylindrical coordinates. The Hue angle is defined by the formula: $H_{ab}$ = tan-1 (b*/a*), wherein a* and b* are coordinates in the L*a*b* color space. A detailed description of hue angle may be found in M L Gulrajani (Ed.). (2010). *Colour Measurement: Principles, Advances and Industrial Applications.* Cambridge, United Kingdom: Woodhouse Publishing.

Typically, the pigment used in the present stable whitening oral care dentifrice compositions is capable of reflecting sufficient light such that the treated tooth is perceivably whiter than its initial color. In some embodiments, the pigment is colored such that its natural color is within the violet-red to green-blue color. More typically, the pigment is violet or blue, *e.g.,* one of those listed in the Colour Index International. These pigments are listed as pigment violet 1 through to pigment violet 56 and pigment blue 1 through 83. In some embodiments, the pigment violets are pigment violet 1, 1:1, 1:2, 2, 3, 5:1, 13, 19, 23, 25, 27, 31, 32, 37, 39, 42, 44 and 50. In some embodiments, the pigment blues are pigment blue 1, 2, 9, 10, 14, 15, 15:1, 15:2, 15:3, 15:4, 15:6 16, 18, 19, 24:1, 25, 56, 60, 61, 62 and 66. Other suitable pigments are pigment ultramarine blue and ultramarine violet. Typically, the pigment is PigmentBlue 15, more typically PigmentBlue 15:1, 15:2, 15:3, 15:4, 15:5 or 15:6, most typically 15:1.

While blue or violet single pigments are typically used in the present stable whitening oral care dentifrice compositions, the same effect may be achieved through mixing pigments outside of the hue angle range of about 220 degrees to about 320 degrees. The desired hue angle may instead be obtained by mixing a red and green-blue pigment to yield a blue or violet shaded pigment.

In the present stable whitening oral care dentifrice compositions, the amount of pigment in the composition is about 0.01% to about 0.3%, typically from about 0.01% to about 0.1%, and more typically from about 0.01% to 0.08% by weight, such as about 0.075%. The pigment may be uniformly spread throughout the composition or may be dispersed in a second phase such as a stripe or other coextruded second phase. Such "dual phase" compositions have the advantage that the phases may be differently colored, presenting a more visually attractive product to the consumer.

*Zinc*

Zinc is included in the present stable whitening oral care dentifrice compositions as set fourth in claim 1.

*Whitening efficacy*

In some embodiments, the present stable whitening oral care dentifrice compositions have a whitening efficiency which is greater than a whitening efficiency of a comparative composition, which may include the same ingredients as a composition of the present disclosure, except that the comparative composition does not contain zinc. As used herein, the phrase "whitening efficacy" is intended to refer to the amount of change in tooth color. The color change can be measured according to the L*a*b* color scale. The luminance or lightness (L*) value measures brightness and varies from a value of one hundred for perfect white to zero for black, assuming a* and b* are zero. The a* value is a measure of redness when positive, gray when zero and greenness when negative. The b* value is a measure of yellowness when positive, gray when zero and blueness when negative. Generally, teeth appear whiter as: the L* value increases meaning they become brighter, the a* value increases or decreases, depending upon whether the stained teeth have a green tint or red tint prior to whitening, and the b* value decreases meaning they become less yellow. While this is the general relationship for perceived whitening, the b* value might also slightly increase if the magnitude of the increase of the L* value is large enough. Similarly, the L* value might also decrease if the magnitude of the decrease of the b* value is large enough to overshadow the less significant change in L*.

In some embodiments, the whitening index (W*) is used to assess tooth whiteness. The whiteness index is based on the distance of a color value from a nominal white point, represented in CIELAB colour space as L* = 100, a* = 0 and b* = 0, and defined according to the following formula:

$$W^* = [(a^*)^2+(b^*)^2+(L^*-100)^2]^{1/2}.$$

Changes in W* may be used to assess the whitening efficacy of a composition before and after a treatment. The following formula may be used to calculate ΔW*:

$$\Delta W^* = W^*(\text{Treatment}) - W^* (\text{baseline}).$$

Other values which may be used to assess tooth whiteness are described in Joiner et al., "A Review of Tooth Colour and Whiteness", Journal of Dentistry, 2008, 36S:S2-S7.

Whitening efficacy of a composition may be determined by any method known in the art. For example, polished hydroxyapatite discs may be placed in sterile human saliva for 2 hours to allow a pellicle to form. The discs may then be rinsed in water and baseline color measurements made (using, for example, a Minolta chromameter CR300). The discs may then be brushed with (i) a composition of the present disclosure or (ii) a comparative composition. The brushing may be performed using a brushing machine. Following rinsing, the color of the discs may then be remeasured and the change in L*, a* and b* recorded for both treatment (i) and treatment (ii) and the W* and ΔW* values calculated. From a comparison of these data, any whitening efficiency of a composition is readily seen. Other methods for assessing whitening efficacy are described in the Examples, herein below.

*Vehicle*

In some embodiments, a whitening agent, complexed with a polymer or co-polymer, additional, optional, polymers and co-polymers, a pigment and zinc as described herein, are combined with an orally acceptable vehicle to form a dentifrice. Such dentifrices may include a dental tablet, toothpaste (dental cream), tooth powders, a viscous liquid, such as a gel, or any other form known to one of skill in the art. Typically, the dentifrice is a gel.

As used herein, an "orally acceptable vehicle" refers to a material or combination of materials that are safe for use in the stable whitening oral care dentifrice compositions of the present disclosure, commensurate with a reasonable benefit/risk ratio, with which the whitening agent, and other desired active ingredients may be associated while retaining significant efficacy. In some embodiments, the combination of ingredients is acidic to maintain stability of the whitening agent.

In some embodiments, the orally acceptable vehicle is a low water content orally acceptable vehicle and may include any known ingredients or additives. For example, the vehicle may include liquid mixtures of water, glycerin, and sorbitol. In some embodiments, the water content of the stable whitening dentifrice composition is less than about 3%, less than about 2%, less than about 1% or less than about 0.1% w/w.

More typically, the orally acceptable vehicle is substantially anhydrous and comprises a hydrophobic component, such as a polymer. The term "hydrophobic" or "water-insoluble" as applied to polymers and as employed herein refers to an organic polymer, which is substantially non-aqueous, and which has a water solubility of less than one gram per 100 grams of water at 25° C.

In some embodiments, the hydrophobic polymers suitable for use in the present orally acceptable vehicle are known in the art as "siloxane" polymers or "silicone" polymers. Typical silicone-based hydrophobic polymers in accordance with the present disclosure include polyorganosiloxane polymers, such as polydimethylsiloxane.

In some embodiments, the siloxane polymers are in the form of a fluid. The polysiloxane fluids may include those with a viscosity at 25° C of about 1 milliPascal-sec (mPa-s) to about 1000 mPa-s, or about 2 mPa-s to about 500 mPa-s or about 20 mPa-s to about 400 mPa-s. Suitable polysiloxane fluids may be linear or cyclic and may be substituted with a wide variety of substituents. In certain embodiments, the substituents include methyl, ethyl and phenyl substituents. Examples of linear polysiloxane and cyclic polymers in accordance with the present disclosure include dimethicone and cyclomethicone, respectively. Other suitable polysiloxane fluids include polysiloxane polyether copolymers and hydroxy-terminated polydimethyl-siloxane fluids, such as ST-DIMETHICONOL™ 40, SGM 36 and SGM3 from Dow Corning Corporation, Midland, MI. Other suitable commercially available polysiloxane fluids include the DC200 series fluids marketed by Dow Corning Corporation and the AK Fluid series marketed by Wacker-Chemie GmbH, Munchen, Germany. High molecular silicone resins with a polysiloxane blend may also be used, including powdered trimethylsiloxysilicate, for example, which is commercially available from Dow Corning Corporation as 593 fluid or from Wacker Chemie AG, Munich, Germany as Wacker BELSIL® TMS 803. Another commercially available suitable silicone fluid is Q7-9210 from Dow Corning Corporation.

In other embodiments, the hydrophobic component comprises a silicone pressure sensitive adhesive (PSA). PSAs can be produced by condensing a silicone resin and an organosiloxane, such as a polydiorganosiloxane. In some embodiments, the silicone polymers are prepared by mixing a silanol terminated polydiorganosiloxane, such as polydimethyl siloxane, with a silanol-containing silicone resin, whereby the silanol groups of the polydiorganosiloxane undergo a condensation reaction with the silanol groups of the silicone resin so that the polydiorganosiloxane is lightly crosslinked by the silicone resin. A catalyst, for example, an alkaline material, such as ammonia, ammonium hydroxide or ammonium carbonate, can be mixed with the silanol-terminated polydiorganosiloxane and the silicone resin to promote crosslinking. By copolymerizing the silicone resin with the silanol terminated polydiorganosiloxane, a polymer with self-adhering properties and a soft elastomer matrix can be produced.

Suitable PSA polymers in accordance with the present disclosure are described in U.S. Publication No. 2015/0037266 and U.S. Publication No 2005/0038181. Suitable commercially available PSA polymers include BIO-PSA polymers from the Dow Corning Corporation. These PSA polymers are available in three silicone resin to silicone polymer ratios, namely, 65/35 (low tack), 60/40 (medium tack), 55/45 (high tack). Without being bound by theory, it is believed that the variation in the ratio of silicone resin to polydiorganosiloxane results in the different tack properties of the BIO-PSA polymers.

In some embodiments, the hydrophobic component is present at a concentration from about 0% to about 80% by weight of the stable whitening oral care dentifrice composition of the present disclosure, such as about 40% to about 80% by weight, such as about 60% to about 80% by weight. In other embodiments, the hydrophobic component may be present in the instant stable whitening dentifrice composition in an amount ranging from about 15% to about 25% by weight, such as about 16% to about 20% by weight, such as about 18% by weight.

In other embodiments, the present substantially anhydrous orally acceptable vehicle includes an ethylene oxide, propylene oxide block co-polymer, such as one having the formula (ethylene oxide)$_x$-(propylene oxide)$_y$, wherein x is an integer of 80-150, *e.g.,* 100-130, *e.g.,* about 118, and y is an integer of 30-80, *e.g.,* about 60-70, *e.g.,* about 66, having an average molecular weight of greater than 5000, *e.g.,* 8000-13000 Da, *e.g.,* about 9800. In some embodiments, the ethylene oxide, propylene oxide co-polymer is substantially free of an ethylene oxide, propylene oxide block co-polymer of average molecular weight less than 5000 Da. An example of a suitable commercially available ethylene oxide, propylene oxide co-polymer is PLURACARE® L1220 (available from BASF, Wyandotte, Michigan, United States of America).

Other suitable low water content orally acceptable vehicles, which may be incorporated into the stable whitening dentifrice composition of the present disclosure include, for example polyethylene glycol, such as PEG400, PEG600, PEG/PPG copolymers, such as PEG/PPG 38/8 copolymer, and PEG/PPG-1 16/66 copolymer sold as PLURACARER L4370 and PLURACARE® L1220 from BASF, Wyandotte, Michigan, respectively. In some embodiments, polyethylene glycol may be present in the instant stable whitening oral care dentifrice compositions in an amount ranging from about 0% to about 0.01%, about 0.01% to about 60%, about 0% to about 15.0%, such as about 10%, about 6.3%, about 7.5%, or about 15% by weight.

In some embodiments, the low water content orally acceptable vehicles, which may be incorporated into the stable whitening dentifrice composition of the present disclosure, include glycerin. In some embodiments, glycerin may be present in the instant stable dentifrice compositions in an amount ranging from about 0.01% to about 60%, such as about 35% by weight.

In some embodiments, the orally acceptable vehicle is present at a concentration from about 0% to about 80%, by weight, of the stable whitening dentifrice composition of the present disclosure, 0.01% to about 60%, such as about 40% to about 80% by weight, such as about 60% to about 80% by weight. In other embodiments, the orally acceptable vehicle is present in the instant stable whitening dentifrice composition of the present disclosure in an amount ranging from about 15% to about 35% by weight, such as about 16% to about 20% by weight, such as about 18% by weight, such as about 6% to about 7.5% by weight.

In some embodiments, the orally acceptable vehicle further comprises a polymer, which aids in the deposition of a pigment, such as pigment blue ("pigment deposition aid"). Without wishing to be bound by theory, it is believed that a pigment deposition aid works by having affinity for both the pigment and the surface of the teeth, the deposition aid serving as a link between the two.

Pigment deposition aids for use in accordance with the present disclosure are typically high molecular weight polymers, *i.e.* polymers having a molecular weight of 200,000 or greater. Suitable pigment deposition aids include hydroxypropylmethylcellulose polymers, for example, hydroxypropylmethylcellulose polymers having a viscosity at 20 °C of about 3.200 to about 4.800 mPa-s using capillary viscometry. Suitable commercially available hydroxypropylmethylcellulose polymers include WALOCEL® HM 4000PA-2910 from Covestro AG, Leverkusen, Germany. Other suitable pigment deposition aids include those described in European Patent No. 1935395, such as GANTREZ™ polymers.

The pigment deposition aid is incorporated into the instant stable whitening oral care dentifrice compositions in an amount of about 0%, or about 0.01% to about 10%, more typically from about 0.05% to about 5%, and most typically from about 0.1% to about 1% by weight.

In a typical embodiment, the pigment deposition aid is able to enhance the whitening efficacy of a stable whitening oral care dentifrice composition in comparison to a stable whitening oral care dentifrice composition, which does contain a pigment deposition aid, by at least 5% and more typically by at least 25%.

In some embodiments, the viscosity of the instant stable whitening oral care dentifrice compositions is from about 1,000 centipoise (cPs) to about 900,000 cPs, such as about 10,000 cPs to about 100,000 cPs, such as about 50,000 cPs to about 900,000 cPs, such as about 200,000 cPs to about 600,000 cPs.

In some embodiments, the orally acceptable vehicle may further comprise surfactants. In some embodiments, surfactants enhance stability of the formulation, help clean the oral cavity surfaces through detergency, and provide foam upon agitation, e.g., during brushing with a dentifrice composition of the disclosure. Surface active agents generally achieve increased prophylactic action, by thoroughly dispersing the whitening agent throughout the oral cavity. In various embodiments, suitable surface active agents may function as a surface active agent, emulsifier, and/or foam modulator.

Any orally acceptable surfactant, most of which are anionic, nonionic or amphoteric, can be used. Suitable anionic surfactants include without limitation water-soluble salts of $C_{8-20}$ alkyl sulfates, sulfonated monoglycerides of $C_{8-20}$ fatty acids, sarcosinates, taurates and the like. Illustrative examples of these and other classes include sodium lauryl sulfate, sodium cocoyl monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include, without limitation, derivatives of $C_{8-20}$ aliphatic secondary and tertiary amines having an anionic group such as carboxylate, sulfate, sulfonate, phosphate or phosphonate. A suitable example is cocoamidopropyl betaine.

In some embodiments, one or more surfactants may be present in a total amount of from about 1.8% to about 4% w/w. In some embodiments, one or more surfactants may be present in a total amount from about 1.9% to about 2% w/w. In some embodiments, one or more surfactants may be present in a total amount of about 2% w/w.

In some embodiments, the stable whitening oral care dentifrice composition of the present disclosure, optionally, comprises a thickening agent. Any orally acceptable thickening agent can be used, including without limitation carbomers, also known as carboxyvinyl polymers, carrageenans, also known as Irish moss and more particularly carrageenan (iota-carrageenan), high molecular weight polyethylene glycols (such as CARBOWAX™, available from The Dow Chemical Company), cellulosic polymers such as hydroxyethylcellulose, carboxymethylcellulose (CMC) and salts thereof, *e.g.,* CMC sodium, natural gums such as karaya, xanthan, gum arabic and tragacanth, colloidal magnesium aluminum silicate, and colloidal and/or fumed silica and mixtures of the same. In some embodiments, the one or more optional thickening agents are present in a total amount of about 0.1 % to about 90% w/w. In some embodiments, the one or more optional thickening agents are present in a total amount of about 1% to about 50% w/w, such as about 1.75%. In some embodiments, the one or more optional thickening agents are present in a total amount of about 5% to about 35% w/w.

In some embodiments, the present compositions optionally comprise an antioxidant. Acceptable antioxidants include BHA, BHT, vitamin A, carotenoids, vitamin E, flavonoids, polyphenols, ascorbic acid, herbal antioxidants, chlorophyll, melatonin and mixtures thereof. In some embodiments, the one or more antioxidants are optionally present in a total amount of about 0.01% to about 0.1% w/w, such as about 0.03% by weight.

Useful flavoring agents include any material or mixture of materials operable to enhance the taste of the composition. Any orally acceptable natural or synthetic flavoring agent can be used, such as flavoring oils, flavoring aldehydes, esters, alcohols, similar materials, and combinations thereof. Flavoring agents include vanillin, sage, marjoram, parsley oil, spearmint oil, cinnamon oil, oil of wintergreen (methylsalicylate), peppermint oil, clove oil, bay oil, anise oil, eucalyptus oil, citrus oils, fruit oils and essences including those derived from lemon, orange, lime, grapefruit, apricot, banana, grape, apple, strawberry, cherry, pineapple, etc., bean- and nut-derived flavors such as coffee, cocoa, cola, peanut, almond, etc., adsorbed and encapsulated flavorants, and mixtures thereof. Also encompassed within flavoring agents herein are ingredients that provide fragrance and/or other sensory effect in the mouth, including cooling or warming effects. Such ingredients include menthol, menthyl acetate, menthyl lactate, camphor, eucalyptus oil, eucalyptol, anethole, eugenol, cassia, oxanone, x-irisone, propenyl guaiethol, thymol, linalool, benzaldehyde, cinnamaldehyde, N-ethyl-p-menthan-3-carboxamine, N,2,3-trimethyl-2-isopropylbutanamide, 3-1-menthoxypropane-1,2-diol, cinnamaldehyde glycerol acetal (CGA), methone glycerol acetal (MGA) and mixtures thereof.

In some embodiments, one or more flavoring agents are optionally present in a total amount of about 0.01% to about 5% w/w. In some embodiments, one or more flavoring agents are optionally present in a total amount of about 0.05% to about 2% w/w. In some embodiments, one or more flavoring agents are optionally present in a total amount of about 0.1% to about 2.5% w/w. In some embodiments, one or more flavoring agents are optionally present in a total amount from about 0.1 % to about 0.5% w/w. In some embodiments, one or more flavoring agents are optionally present in the total amount of about 1.4% w/w.

Sweeteners among those useful herein include orally acceptable natural or artificial, nutritive or non-nutritive sweeteners. Such sweeteners include dextrose, polydextrose, sucrose, maltose, dextrin, dried invert sugar, mannose, xylose, ribose, fructose, levulose, galactose, corn syrup (including high fructose corn syrup and corn syrup solids), partially hydrolyzed starch, hydrogenated starch hydrolysate, sorbitol, mannitol, xylitol, maltitol, isomalt, aspartame, neotame, saccharin and salts thereof, sucralose, dipeptide-based intense sweeteners, cyclamates, dihydrochalcones and mixtures thereof. Some embodiments optionally comprise one or more sweeteners. In some embodiments, the one or more optional sweeteners are present in a total amount from about 0.005% to about 5% w/w. In some embodiments, the one or more optional sweeteners are present in a total amount from about 0.01 to about 1 % w/w.

pH modifying agents among those useful herein include acidifying agents to lower pH, basifying agents to raise pH and buffering agents to control pH within a desired range. For example, one or more compounds selected from acidifying, basifying and buffering agents can be included to provide a pH of 2 to 10, or in various embodiments from 2 to 8, from 3 to 9, from 4 to 8, from 5 to 7, from 6 to 10, and from 7 to 9. Any orally acceptable pH modifying agent can be used, including without limitation carboxylic, phosphoric and sulfonic acids, acid salts (e.g., monosodium citrate, disodium citrate,. monosodium malate, etc.), alkali metal hydroxides such as sodium hydroxide, carbonates such as sodium carbonate, bicarbonates, sesquicarbonates, borates, silicates, phosphates (e.g., monosodium phosphate, trisodium phosphate, pyrophosphate salts, etc.), imidazole and mixtures thereof. One or more pH modifying agents are optionally present in a total amount effective to maintain the composition in an orally acceptable pH range.

Colorants, mouth-feel agents and/or others additives may also be included, if desired, in the present compositions.

*Other Active Ingredients*

The stable whitening oral care dentifrice compositions of the present disclosure optionally comprise one or more further active material(s), which is operable for the prevention or treatment of a condition or disorder of hard or soft tissue of the oral cavity, the prevention or treatment of a physiological disorder or condition, or to provide a cosmetic benefit.

Some embodiments of the present disclosure comprise a dental abrasive or combination of dental abrasive agents. As used herein, the term "abrasive" or "abrasive agent" also includes materials commonly referred to as "polishing agents." Any orally acceptable abrasive can be used, but typically, type, fineness (particle size) and amount of abrasive should be selected so that tooth enamel is not excessively abraded in normal use of the composition. Suitable abrasives include without limitation silica (in the form of silica gel, hydrated silica or precipitated silica), alumina, insoluble phosphates, calcium carbonate, resinous abrasives such as urea-formaldehyde condensation products and the like.

Among insoluble phosphates useful as abrasives are orthophosphates, polymetaphosphates and pyrophosphates. Illustrative examples are dicalcium orthophosphate dihydrate, calcium pyrophosphate, n-calcium pyrophosphate, tricalcium phosphate, calcium polymetaphosphate and insoluble sodium polymetaphosphate.

Average particle size of an abrasive, if present, is generally about 0.1 to about 30 $\mu$m for example about 1 to about 20 $\mu$m or about 5 to about 15 $\mu$m. In some embodiments, one or more abrasives are present in an amount of about 0.01 % to about 40% w/w, such as 0.01% to about 10%, such as 1.75%. In some embodiments, the abrasive is calcium pyrophosphate. In some embodiments, the calcium pyrophosphate is present in an amount from about 5% to about 50% w/w, such as about 20%.

In various embodiments of the present disclosure, the stable whitening oral care dentifrice composition comprises an anticalculus agent. Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. In some embodiments, the anticalculus agent is present in an amount of about 0.1% to about 30% w/w. In some embodiments, the stable whitening dentifrice composition comprises a mixture of anticalculus agents. In some embodiments, tetrasodium pyrophosphate (TSPP) and sodium tripolyphosphate (STPP) are used as the anticalculus agents. In some embodiments, the anticalculus agent comprises about 0.01% to about 5% w/w TSPP, such as about 2%.

Another desirable component of the present compositions is a synthetic anionic polymeric polycarboxylate (SAPP), which acts as a stabilizer for the polyphosphate anti-tartar agent and which may help to block access of painful or pain-causing materials, such as sugars, to the tooth nerves.

In some embodiments, the present stable whitening oral care dentifrice composition optionally comprises a source of fluoride ions. In some embodiments, the source of fluoride ions is selected from: fluoride, monofluorophosphate (MFP), and fluorosilicate salts. In some embodiments, one or more fluoride ion-releasing compounds are optionally present in an amount providing a total of 100 to 20,000 ppm, 200 to 5,000 ppm, or 500 to 2,500 ppm, fluoride ions. If present, the amount of fluoride in the present composition ranges from about 0.1% to 1.1%, typically about 1.1%.

The compositions also may include a stannous ion or a stannous ion source to mitigate calcium loss. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of about 0.01% to about 10%, for example about 0.1% to about 7% or about 1% to about 5%.

The compositions of the present disclosure may optionally comprise an antimicrobial (e.g., antibacterial) agent in addition to the zinc salts described herein. An illustrative list of useful antibacterial agents is provided in U.S. Patent No. 5,776,435 to Gaffar et al.. One or more antimicrobial agents in addition to the zinc salts described herein are optionally present in an antimicrobial effective total amount, typically about 0.05% to about 10%, for example about 0.1% to about 3%.

Methods

In various embodiments, the present disclosure provides methods to whiten an oral surface in a human or animal subject comprising contacting a tooth surface with a stable whitening dentifrice composition of the present disclosure. As used herein "animal subject" includes non-human mammals such as canines, felines and horses. The stable whitening dentifrice composition is contacted with an oral surface of the mammalian subject to thereby whiten teeth in a highly efficacious manner.

In various embodiments, the stable whitening dentifrice composition prepared in accordance with the present disclosure may be applied regularly to an oral surface, for example on a daily basis, at least one time daily for multiple days, or alternately every second or third day. In some embodiments, the stable whitening dentifrice composition is applied to the oral surfaces from 1 to 3 times daily, for at least 2 weeks up to 8 weeks, from four months to three years, or more up to a lifetime.

In some embodiments, the stable whitening dentifrice composition, such as a gel, may be applied directly to the teeth using a delivery device, such as a pen, e.g., a COLGATE® whitening pen or a COLGATE® ACTIS™ whitening pen, Colgate-Palmolive Company, New York, NY, a liquid stick having an applicator, such as a felt tip, brush, roller ball, or non-woven pad, sufficient to effect whitening. In some embodiments, the stable whitening dentifrice composition of the present disclosure is maintained on the surface of the tooth for a plurality of minutes.

In some embodiments, the composition is maintained on the surface of a tooth for from about 1 minute to about 8 hours. In some embodiments, the composition is maintained on the surface of a tooth for from about 5 minutes to about 4 hours. In some embodiments, the composition is maintained on the surface of a tooth for from about 10 minutes to about 120 minutes. In some embodiments, the composition is maintained on the surface of a tooth for from about 15 minutes to about 60 minutes. In some embodiments, the composition is maintained on the surface of a tooth for from about 20 minutes to about 45 minutes.

Some embodiments provide a method wherein a delivery device, such as a whitening pen is stored within an oral care implement, such as a toothbrush. In some embodiments, the delivery device, such as a whitening pen is removed from the oral care implement prior to application of the composition to the tooth. In some embodiments, the composition is applied to the tooth after brushing with the oral care implement.

The examples and other embodiments described herein are exemplary and not intended to be limiting in describing the full scope of compositions and methods of this disclosure. Equivalent changes, modifications and variations of specific embodiments, materials, compositions and methods may be made within the scope of the present disclosure, with substantially similar results.

**EXAMPLES**

Example 1. Stability of the Present Compositions

A whitening dentifrice according to the present disclosure containing a low concentration of hydrogen peroxide (0.1 wt.%) in the form of 0.55 wt% cross-linked PVP complexed with hydrogen peroxide, pigment blue 15 and zinc was prepared by mixing the ingredients of Table 1, below (Example 1). A second 0.1 wt% hydrogen peroxide whitening dentifrice was also prepared, which contained the same ingredients as shown in Table 1, except that zinc and pigment blue 15 were excluded (Comparative Example A).

Each of the above-described compositions was aged for three months at 40 °C (75% humidity) in an oven. Stability of the peroxide was analyzed at three time points. The results shown in Table 2 depict the degradation of hydrogen peroxide in the present whitening dentifrice composition and Comparative Composition A. As is evident from Table 2, hydrogen peroxide is stable in the whitening oral care dentifrice composition of the instant disclosure.

Table 1.

| Low peroxide toothpaste formulation with zinc and pigment blue | | |
|---|---|---|
| Ingredient | Range (wt.%) | Example 1 |
| Humectant | 0.01-60 | 35.41 |
| PLURACARE® L1220 | 0.01-10 | 7.5 |
| Propylene Glycol | 0.01-60 | 15 |
| Polyethylene Glycol 600 | 0-15 | 6.31 |
| Cross-linked PVP complexed with hydrogen peroxide | 0.05-0.55 | 0.55 |
| Calcium pyrophosphate | 0.01-40 | 20 |
| Fluoride | 0-1.1 | 1.1 |
| Cross-linked PVP | 0-10 | 5.75 |
| Fumed silica | 0.01-10 | 1.75 |
| Sodium lauryl sulfate | 0-4 | 2 |
| Flavor | 0.01-4.0 | 1.4 |
| Water | 0-3 | 0 |
| TSPP | 0.01-5.00 | 2 |
| BHT | 0.01-0.1 | 0.03 |
| Pigment Blue 15 | 0.01-0.08 | 0.075 |
| Phosphoric acid | 0.01-3 | 0.2 |
| Zinc oxide | 0.2-2 | 1 |
| **Total** | **100** | **100** |

**Table 2.**

| Degradation of hydrogen peroxide | | | |
|---|---|---|---|
| Formulation | Initial % Oxygen | 1 Month 40 °C | 3 Month 40 °C |
| (0.1%) Hydrogen Peroxide Toothpaste with Pigment Blue and Zinc (Example 1) | 0.10 | 0.09 | 0.07 |
| (0.1%) Hydrogen Peroxide Toothpaste (Comparative Composition A) | 0.10 | 0.09 | 0.08 |

Example 2. Whitening Efficacy

The whitening efficacy of the stable whitening oral care dentifrice composition of the present disclosure and the Comparative Composition A as described above were assessed *in vitro* using brushing experiments. In addition, a second comparative composition was also assessed for whitening efficacy (Comparative Composition B). Comparative Composition B contained the same ingredients shown in Table 1 including 0.1% hydrogen peroxide. However, Comparative Composition B did not contain zinc.

Specifically, artificially stained bovine teeth were polished with prophylaxis paste to achieve similar initial lightness values among the samples. Baseline lightness values of the teeth were assessed with a spectrophotometer. Six bovine teeth were then mounted onto each of three trays. Three slurries were prepared containing i) a 1:1 (w/w) of the present stable whitening oral care dentifrice composition and deionized water, ii) a 1:1 (w/w) of Comparative Composition A and deionized water and iii) a 1:1 (w/w) of Comparative Composition B and deionized water. 25 grams of each slurry were added to each tray. The stained bovine teeth in each tray were brushed for two minutes with slurry i), ii) or iii) at 120 strokes/minute. The teeth were then rinsed with 100 milliliters of deionized water. Fourteen (14) brushing cycles were performed in total. The whiteness of the teeth was evaluated for L*, a* and b* values with a spectrophotometer and the whiteness values (W*) as described herein were calculated. Changes in W* values were also determined and are described in Table 3.

**Table 3**

| Whitening Efficacy | | |
|---|---|---|
| | $\Delta W^*$ | $\Delta b$ |
| (0.1%) Hydrogen Peroxide Toothpaste with pigment blue and zinc (Example 1) | -10 | -0.4 |
| (0.1%) Hydrogen Peroxide (Comparative Composition A) | -7.9 | -0.1 |
| (0.1%) Hydrogen Peroxide Toothpaste with pigment blue (Comparative Composition B) | -8.5 | -0.4 |

As shown in Table 3, the stable whitening oral care dentifrice composition Example 1 of the present disclosure, which contains pigment blue 15, zinc and 0.1% hydrogen peroxide exhibited a $\Delta b$ value of -0.4, indicating the amount of blue or yellow coloring of the teeth. This value was comparable to the $\Delta b$ value observed for Comparative Composition B (-0.4), which contains 0.1% hydrogen peroxide and pigment blue, but does not contain zinc. The $\Delta b$ value for Comparative Composition A, which contains 0.1% hydrogen peroxide, but does not contain either pigment blue or zinc, was substantially lower (-0.1) than either the present whitening dentifrice composition or Comparative Composition B.

Surprisingly, however, the stable whitening oral care dentifrice composition of the present disclosure exhibited greater whitening efficacy ($\Delta W^* = -10$) than that of Comparative Composition A ($\Delta W^* = -7.9$) or Comparative Composition B ($\Delta W^* = -8.5$). Accordingly, the present stable whitening oral care dentifrice composition results in an improved whitening efficacy when compared to hydrogen peroxide-containing dentifrice compositions, which do not contain a combination of pigment blue with or without zinc.

**Claims**

1. A stable whitening dentifrice composition comprising:

a whitening complex, wherein the whitening complex comprises (a) a whitening agent being hydrogen peroxide and (b) a cross-linked vinyl lactam based polymer being a cross-linked polyvinylpyrrolidone;

a blue pigment having a blue to blue-violet color with a hue angle in the CIELAB system ranging from 200 degrees to 320 degrees;
a zinc salt in an amount ranging from 0.5% to 2% by weight, said zinc salt comprising zinc oxide;
and a linear polyvinylpyrrolidone or an additional cross-linked polyvinylpyrrolidone, wherein the additional cross-linked polyvinylpyrrolidone is not complexed with the whitening agent;
an orally acceptable vehicle.

2. The stable whitening dentifrice composition of claim 1, wherein a total amount of hydrogen peroxide by weight of the composition is 0.05% to 4%.

3. The stable whitening dentifrice composition of any of the preceding claims, wherein the blue pigment has a hue angle in the CIELAB system ranging from 250 to 290 degrees.

4. The stable whitening dentifrice composition of any of the preceding claims, wherein the composition is a gel.

5. The stable whitening dentifrice composition of any of the preceding claims, wherein the orally acceptable vehicle is anhydrous.

6. The stable whitening dentifrice composition of any of the preceding claims, wherein the orally acceptable vehicle comprises an ethylene oxide, propylene oxide block co-polymer of average molecular weight greater than 5000 Da.

7. The stable whitening dentifrice composition of claim 7, wherein the ethylene oxide, propylene oxide block co-polymer comprises (ethylene oxide)$_x$-(propylene oxide)$_y$, wherein x is an integer of 80-150 and y is an integer of 30-80, having an average molecular weight of greater than 5000 Da.

8. The stable whitening dentifrice composition of any of the preceding claims, wherein the composition further comprises an abrasive.

9. The stable whitening dentifrice composition of any of the preceding claims, wherein the composition further comprises a hydroxypropylmethylcellulose polymer having a viscosity at 20 °C of 3.200 to 4.800 mPa.s.

10. The stable whitening dentifrice composition of any of the preceding claims for use in a method for whitening a tooth surface in a human or animal, wherein the method comprises contacting the stable whitening dentifrice composition with the tooth surface.

**Patentansprüche**

1. Stabile bleichende Zahnpastenzusammensetzung, umfassend:

einen bleichenden Komplex, wobei der bleichende Komplex (a) ein Bleichungsmittel, das Wasserstoffperoxid ist, und (b) ein vernetztes Polymer auf Vinyllactam-Basis, das ein vernetztes Polyvinylpyrrolidon ist, umfasst;
ein blaues Pigment mit einer blauen bis blau-violetten Farbe mit einem Farbtonwinkel im CIELAB-System von 200 Grad bis 320 Grad;
ein Zinksalz in einer Menge von 0,5 bis 2 Gew.-%, wobei das Zinksalz Zinkoxid umfasst;
und ein lineares Polyvinylpyrrolidon oder ein zusätzliches vernetztes Polyvinylpyrrolidon, wobei das zusätzliche vernetzte Polyvinylpyrrolidon nicht mit dem Bleichmittel komplexiert ist;
ein oral verträgliches Vehikel.

2. Stabile bleichende Zahnpastenzusammensetzung nach Anspruch 1, wobei die Gesamtmenge an Wasserstoffperoxid, bezogen auf das Gewicht der Zusammensetzung, 0,05% bis 4% beträgt.

3. Stabile bleichende Zahnpastenzusammensetzung nach einem beliebigen der vorhergehenden Ansprüche, wobei das blaue Pigment einen Farbtonwinkel im CIELAB-System im Bereich von 250 bis 290 Grad aufweist.

4. Stabile bleichende Zahnpastenzusammensetzung nach einem beliebigen der vorhergehenden Ansprüche, wobei die Zusammensetzung ein Gel ist.

**5.** Stabile bleichende Zahnpastenzusammensetzung nach einem beliebigen der vorhergehenden Ansprüche, wobei das oral verträgliche Vehikel wasserfrei ist.

**6.** Stabile bleichende Zahnpastenzusammensetzung nach einem beliebigen der vorhergehenden Ansprüche, wobei das oral verträgliche Vehikel ein Ethylenoxid-Propylenoxid-Blockcopolymer mit einem durchschnittlichen Molekulargewicht von mehr als 5000 Da umfasst.

**7.** Stabile bleichende Zahnputzmittelzusammensetzung nach Anspruch 7, wobei das Ethylenoxid-Propylenoxid-Blockcopolymer (Ethylenoxid)$_x$-(Propylenoxid)$_y$ umfasst, wobei x eine ganze Zahl von 80-150 und y eine ganze Zahl von 30-80 ist, mit einem durchschnittlichen Molekulargewicht von mehr als 5000 Da.

**8.** Stabile bleichende Zahnpastenzusammensetzung nach einem beliebigen der vorhergehenden Ansprüche, wobei die Zusammensetzung weiterhin ein Abrasiv umfasst.

**9.** Stabile bleichende Zahnpastenzusammensetzung nach einem beliebigen der vorhergehenden Ansprüche, wobei die Zusammensetzung weiterhin ein Hydroxypropylmethylcellulosepolymer mit einer Viskosität bei 20° C von 3.200 bis 4.800 mPa.s umfasst.

**10.** Stabile bleichende Zahnpastenzusammensetzung nach einem beliebigen der vorhergehenden Ansprüche zur Verwendung in einem Verfahren zum Bleichen einer Zahnoberfläche bei einem Menschen oder einem Tier, wobei das Verfahren das Inkontaktbringen der stabilen bleichenden Zahnpastenzusammensetzung mit der Zahnoberfläche umfasst.

**Revendications**

**1.** Composition de dentifrice blanchissant stable comprenant :

un complexe blanchissant, dans lequel le complexe blanchissant comprend (a) un agent de blanchiment étant du peroxyde d'hydrogène et (b) un polymère à base de vinyl lactame réticulé étant une polyvinylpyrroiidone réticulée ;
un pigment bleu ayant une couleur bleu à bleu-violet avec un angle de teinte dans le système CIELAB allant de 200 degrés à 320 degrés ;
un sel de zinc en une quantité allant de 0,5 % à 2 % en poids, ledit sel de zinc comprenant de l'oxyde de zinc ;
et une polyvinylpyrrolidone linéaire ou une polyvinylpyrrolidone réticulée supplémentaire, la polyvinylpyrrolidone réticulée supplémentaire n'étant pas complexée avec l'agent de blanchiment ;
un véhicule oralement acceptable.

**2.** Composition de dentifrice blanchissant stable selon la revendication 1, dans laquelle la quantité totale de peroxyde d'hydrogène en poids de la composition est de 0,05 % à 4 %.

**3.** Composition de dentifrice blanchissant stable selon l'une quelconque des revendications précédentes, dans laquelle le pigment bleu a un angle de teinte dans le système CIELAB allant de 250 à 290 degrés.

**4.** Composition de dentifrice blanchissant stable selon l'une quelconque des revendications précédentes, dans laquelle la composition est un gel.

**5.** Composition de dentifrice blanchissant stable selon l'une quelconque des revendications précédentes, dans laquelle le véhicule oralement acceptable est anhydre.

**6.** Composition de dentifrice blanchissant stable selon l'une quelconque des revendications précédentes, dans laquelle le véhicule oralement acceptable comprend un copolymère séquencé d'oxyde d'éthylène et d'oxyde de propylène d'un poids moléculaire moyen supérieur à 5 000 Da.

**7.** Composition de dentifrice blanchissant stable selon la revendication 7, dans laquelle le copolymère séquencé d'oxyde d'éthylène et d'oxyde de propylène comprend (oxyde d'éthylène)$_x$-(oxyde de propylène)$_y$, dans lequel x est un nombre entier de 80 à 150 et y est un nombre entier de 30 à 80, ayant un poids moléculaire moyen supérieur à 5 000 Da.

8. Composition de dentifrice blanchissant stable selon l'une quelconque des revendications précédentes, dans laquelle la composition comprend en outre un abrasif.

9. Composition de dentifrice blanchissant stable selon l'une quelconque des revendications précédentes, dans laquelle la composition comprend en outre un polymère d'hydroxypropylméthylcellulose ayant une viscosité à 20 °C entre 3,200 et 4,800 mPa.s.

10. Composition de dentifrice blanchissant stable selon l'une quelconque des revendications précédentes pour une utilisation dans un procédé de blanchiment d'une surface dentaire chez un humain ou un animal, dans laquelle le procédé comprend la mise en contact de la composition de dentifrice blanchissant stable avec la surface des dents.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015099642 A1
- US 2007122361 A1
- WO 2006079342 A1
- US 5122370 A
- WO 2015099642 A
- US 20150037266 A
- US 20050038181 A
- EP 1935395 A
- US 5776435 A, Gaffar

### Non-patent literature cited in the description

- **KRAUS et al.** whole saliva ... decomposed more than 90% of the peroxide. *Journal of Bacteriology,* 1957, vol. 76 (727), 733
- *Analytica Chimica Acta,* 2002, vol. 455, 187-191
- Colour Measurement: Principles, Advances and Industrial Applications. United Kingdom: Woodhouse Publishing, 2010
- **JOINER et al.** A Review of Tooth Colour and Whiteness. *Journal of Dentistry,* 2008, vol. 36S, S2-S7